# EUROPEAN PATENT APPLICATION

(11) **EP 4 424 832 A1**
(43) Date of publication of application: **04.09.2024**
(21) Application number: 22886204.1
(22) Date of filing: 25.10.2022
(51) Int. Cl.: C12N 15/85, A61P 35/00, A61K 48/00

(54) **GENE THERAPY WITH THE GENES HOKD AND LDRB FOR CANCER TREATMENT**

(30) Priority: 25.10.2021 ES 202131001
(71) Applicant: Universidad de Granada, 18071 Granada (ES); Servicio Andaluz de Salud, 41071 Sevilla (ES)
(72) Inventor: BOULAIZ TASSI, Houria, 18071 Granada (ES); MARCHAL CORRALES, Juan Antonio, 18071 Granada (ES); JIMÉNEZ MARTÍNEZ, Yaiza, 41071 Sevilla (ES); GRIÑAN LISON, Carmen, 18071 Granada (ES)
(74) Representative: San Martin Alarcia, Esther
(86) International application number: PCT/ES2022/070693
(87) International publication number: WO 2023/073270

(57) **Abstract**

The invention relates to the RNA or DNA polynucleotides hokD and IdrB isolated for use in medicine, particularly in gene therapy for cancer treatment. The invention also describes compositions comprising said polynucleotides or related gene constructs, and a method for treating proliferative disorders, preferably cancer.

## Description

### TECHNICAL FIELD

The present invention belongs to the field of gene therapy. More specifically, it describes the use of HOKD and LDRB toxins, with application in the field of gene therapy and especially in the treatment of cancer.

### STATE OF THE ART

Cancer is one of the main causes of death worldwide, only surpassed by some cardiovascular diseases. Moreover, it is estimated that cancer cases and deaths will grow exponentially over the next few years. Among cancers with more incidences and more mortality are lung and bronchus cancer, prostate cancer, breast cancer, colorectal cancer and cervical cancer.

Focusing on women, breast and cervical cancer are in the ranking of most common cancers. In 2018, the number of cases was 2.1 million cases of breast cancer and almost 0.6 million cases of cervical cancer. As regards mortality, cervical cancer caused more than 300 thousand deaths in 2018 and breast cancer produced more than 40 thousand deaths in 2019. Best tests to detect these types of cancer are the papinocolau test in case of cervical cancer and mammography for breast cancer. Despite the decrease in cases in early stages, in advanced stages these cancers are lethal, especially cervical cancer.

The complexities that underlie cancer reside in mechanisms causing the development of the tumor, its staging, diagnostic, prognostic and difficult treatment. Most known treatments are surgery, chemotherapy and radiotherapy. However, in the majority of the cases these treatments are not as effective as it is wanted, being able to cause the relapse in the patient. Moreover, in cervical cancer, surgical removal of the uterus causes infertility in the patient, which gives an idea of the aggressiveness of these therapies. Therefore, researches in new therapies against cancer, more effective and less aggressive, are expanding. Among these new therapies are immunotherapy, hormonotherapy or gene therapy, one of the most interesting and current therapies. It consists in the utilization of nucleic acids such as DNA or RNA for the purpose of curing, treating or preventing human diseases, including cancer. To achieve this, the aim is to replace a defective gene with a functional one, introduce a missing gene or even a therapeutic one using different tools, for example, viral or non-viral vectors. These types of therapies can be classified according to the way the vector is administered to the patient (*in vivo* or ex *vivo*), the vehicle used and its characteristics (integrating/stable or non-integrating/transient), among others [1].

Within gene therapy based on suicide genes there are two main types; direct therapy, where a gene responsible for the production of a toxin is introduced in order to destroy the cell from within, and indirect therapy, where the gene introduced is responsible for the production of an enzyme whose role is the transformation of an administered substance or prodrug into a drug. The present invention is framed within direct gene therapy, which presents fewer limitations than indirect therapy, such as the need for a prodrug, the problems of toxicity of the prodrug or the limited bioavailability of the prodrug [**2**].

Gene therapy offers a great advantage over other therapies.

Tissue-specific promoters refer to those promoters specifically active in certain tissues; cancer-specific promoters to those that are active in various types of cancer but not in any specific tissue/tumor; and tumor-specific promoters are those functional in different types of cancer cells having variations in their activity depending on the tumor [3]. One of the most important advantages of gene therapy is the possibility of targeting cancer cells **using tissue-specific promoters.**

There are numerous studies related to the use of these promoters in suicide gene therapy, investigating, for example, the effect of the telomerase promoter (hTERT), which was the first gene to be classified as cancer-specific; epidermal growth factor receptor (EGFR) promoter; survival promoter; breast cancer promoters 1 (BRCA1) and BRCA2, which are tumor-specific; among others [3].

Survivin is a protein that acts as a cell cycle modulator by inhibiting apoptosis when overexpressed in the G2/M phase of the cycle. Therefore, it is mildly expressed in adult non-tumor tissues such as primitive hematopoietic cells, vascular endothelial cells, colonic and gastrointestinal mucosal cells, etc. [**4**]. It is overexpressed in **cancer stem cells ("CSCs").** In a recent study based on the mechanisms underlying teratoma formation, survivin was overexpressed in human embryonic stem cells and teratoma [**5**]. In addition, other studies have focused on survivin overexpression in different CSCs [**6**]. Thus, survivin could be used as a tumor cell line-specific promoter, directing gene expression only in target cells. [**7**].

The use of genes encoding toxic proteins has been proposed as a promising tool for cancer gene therapy. Its main advantage is the ability to kill even dormant tumor cells, whereas classical genes used in conventional suicide gene therapy only target rapidly dividing cells by disrupting DNA synthesis. Different bacterial suicide genes such as gef toxin, streptolysin O toxin, diphtheria toxin and pseudomonas exotoxin with potent antitumor effect have been described in the last decades [**8-11**]. In this context, previous investigations confirmed the role of the ***IdrB* gene as a suicide gene in eukaryotic cells.** This gene belongs to the Idr/rdl gene family present in the *Escherichia coli* genome. *LdrB* encodes a small toxin whose overexpression causes cell death. The function of the toxin is nucleoid condensation, preventing transcription and translation, leading to loss of cell viability and death [**12**]. On the other hand, ***hokD*** is one of the five homologs of the hok gene located in the R1 plasmid of *E*. *coli* and encodes a 52 amino acid transmembrane protein that alters the cell membrane and causes **inhibition of tumor proliferation [13].**

It is now well known that cancer is considered a group of different disorders due to the enormous tumor heterogeneity that exists. This heterogeneity can be explained, among other reasons, by the CSC concept. It means that the tumor is composed in minor proportion by CSCs, which are responsible for the maintenance and proliferation of the tumor, being the only cell type that can self-renew and differentiate into non-stem cells. Thus, **CSC could be the cause of resistance to chemotherapy, the source of tumor cells and responsible for metastasis [14-16].**

The different treatments commonly used in cancer have some drawbacks and generate a long list of side effects for the patient. For example, the toxicity of chemotherapy and its severe side effects can cause chronic damage. Early side effects can be seen in the bone marrow, hair, skin, blood, kidneys, among others. Taking this into account, it can be said that the main problem of cancer therapies today is the loss of the specific target of the tumor. All this calls for new, more innovative and less aggressive therapeutic strategies. In this context, **gene therapy** represents a good approach. Commonly used systems for suicide gene therapy are Herpes Simplex Virus thymidine kinase (TK) and ganciclovir, cytosine deaminase (CD) and 5-fluorocytosine (5-FC), purine nucleoside phosphorylase and 6-methylpurine deoxyriboside, among others [**17**].

Some suicide gene therapies, particularly indirect ones, could cause extra damage or further complications due to the possible toxicity of the prodrugs considering that they require high concentrations of prodrugs. **Direct suicide therapy** seems to be a good alternative.

### BRIEF DESCRIPTION OF THE INVENTION

In the present invention, the **antitumor efficacy of *IdrB* and *hokD* genes under the control of different promoters (induced or tissue-specific promoters)** is demonstrated in **different cell lines such as HeLa (cervical cancer) and MCF-7 (breast cancer).**

In order to evaluate the **antiproliferative capacity** of the chosen suicide genes and the specificity of the promoters used, two- and three-dimensional cultures of cervical (HeLa) and breast (MCF-7) cell lines were performed. The **effect of the *hokD* and *IdrB* genes,** on the commercial inducible promoter TRE3G-Dox used as a control promoter and on the **survivin** promoter, a cancer-specific promoter, was tested in order to achieve a **targeted therapy.**

**A first aspect of the invention** is constituted by a genetic construct, hereinafter referred to as **"genetic construct of the invention",** comprising a nucleotide sequence corresponding to a gene expression system or vector comprising at least one RNA or DNA polynucleotide of the *hokD* gene and of a proliferation inhibitor gene, operatively linked to at least one promoter that directs transcription of said nucleotide sequence, and to other sequences necessary or appropriate for transcription and their regulation appropriate in time and place for transcription *in vitro* or *in vivo.* When we speak of the *hokD* Gene we refer to an isolated RNA or DNA polynucleotide that has an identity of at least 80%, preferably 85%, 90%, 95% and most preferably 99% with the polynucleotide sequence of the *hokD* gene (SEO ID NO: 1). More preferably the polynucleotide is the *hokD* gene, of nucleotide sequence SEO ID NO: 1.
**SEQ ID NO: 1** 
ATGAAGCAGCAAAAGGCGATGTTAATCGCCCTGATCGTCATCTGTTTAACCGTCATAGTGACGGC
ACTGGTAACGAGGAAAGACCTCTGCGAGGTACGAATCCGAACCGGCCAGACGGAGGTCGCTGT
CTTCACAGCTTACGAACCTGAGGAGTAA
 isolated RNA or DNA polynucleotide, hereinafter first polynucleotide of the invention, capable of translating into an amino acidic sequence comprising a peptide having at least 90% identity to the amino acidic sequence of the HOKD protein (SEQ ID NO: 3). More preferably, it refers to an isolated RNA or DNA polynucleotide capable of translation into an amino acid sequence comprising a peptide having at least 95%, 96%, 97%, 98% or 99% identity to the amino acid sequence of the HOKD protein.
**SEQ ID NO: 3**
   MKQQKAMLIALIVICLTVIVTALVTRKDLCEVRIRTGQTEVAVFTAYEPEE

**In a preferred embodiment of this aspect** of the invention, the proliferation inhibitor gene is *IdrB.*

When we speak of the IdrB Gene we refer to an isolated RNA or DNA polynucleotide, which has an identity of at least 80%, preferably 85%, 90%, 90%, 95% and most preferably 99% with the polynucleotide sequence of the IdrB gene (SEQ ID NO: 2). More preferably the polynucleotide is the IdrB gene, of nucleotide sequence SEQ ID NO: 2.

SEQ ID NO: 2
ATGACGCTCGCGCAGTTTGCCATGACTTTCTGGCACGACCTGGCGGCACCGATCCTGGCGGGAA
TTATTACCGCAGCGATTGTCGGCTGGTGGCGTAACCGGAAGTAA

Isolated RNA or DNA polynucleotide, hereinafter second polynucleotide of the invention, capable of translating into an amino acidic sequence comprising a peptide having at least 90% identity to the amino acidic sequence of the LDRB protein (SEQ ID NO: 4). More preferably it refers to an isolated RNA or DNA polynucleotide capable of translation into an amino acid sequence comprising a peptide having at least 95%, 96%, 97%, 98% or 99% identity to the amino acid sequence of the LDRB protein. **SEQ ID NO: 4** MTLAQFAMTFWHDLAAPILAGIITAAIVGWWRNRK

**In a most preferred embodiment of this aspect** of the invention, the transcription-directing promoter is selected from the list consisting of telomerase promoter, epidermal growth factor receptor promoter, transferrin receptor promoter, carcinoembryonic antigen promoter and survivin promoter or any combination thereof.

**In an even more preferred embodiment of this aspect** the promoter directing transcription is the survivin promoter.

**Another aspect of the invention** relates to a cell, hereinafter **"cell of the invention",** comprising the genetic construct of the invention.

**Another aspect of the invention** describes a composition, hereinafter **"composition of the invention",** comprising the genetic construct of the invention and/or the cell of the invention. **Another aspect of the invention** is based on the genetic construct of the invention, the cell of the invention, and/or the composition of the invention, for use as a medicament.

**Another aspect of the invention** is based on the genetic construct of the invention, the cell of the invention, and/or the composition of the invention for the prevention, amelioration, alleviation or treatment of a proliferative disease.

**In a preferred embodiment** of this aspect of the invention, the proliferative disease is cancer.

**In a more preferred embodiment** the cancer is characterized by presenting cancer stem cells. Preferably, it is a cancer where the stem cells give rise to metastasis, resistance, recurrence or relapse of the disease.

**In other preferred embodiments** the proliferative disease is breast cancer or cervical cancer.

**Another aspect of the invention** is the use as a medicament for the prevention, amelioration, alleviation, or treatment of breast cancer of the genetic construct of the invention, the cell of the invention, and/or the composition of the invention, in combination with any of the drugs selected from the list consisting of: Trastuzumab, Trastuzumab deruxtecan, Pertuzumab, Ado-trastuzumab emtansine, Sacituzumab govitecan, Tucatinib, Neratinib, Lapatinib, Palbociclib, Ribociclib, Abemaciclib, Alpelisib, Everolimus, Olaparib, Talazoparib, and Atezolizumab or any combinations thereof.

**Another aspect of the invention** is the use as a medicament for the prevention, amelioration, alleviation, or treatment of cervical cancer of the genetic construct of the invention, the cell of the invention, and/or the composition of the invention, in combination with any of the drugs selected from the list consisting of: Cisplatin, Bevacizumab, Pembrolizumab, Carboplatin, Tisotumab vedotin, Gemcitabine, Ifosfamide, Irinotecan, and Paclitaxel or any combination thereof.

Throughout the description and the claims the word "comprises" and variants thereof are not intended to exclude other technical features, additives, components or steps. To those skilled in the art, other objects, advantages and features of the invention will be apparent in part from the description and in part from the practice of the invention. The following examples and drawings are provided by way of illustration, and are not intended to be limiting of the present invention.

### DESCRIPTION OF THE FIGURES

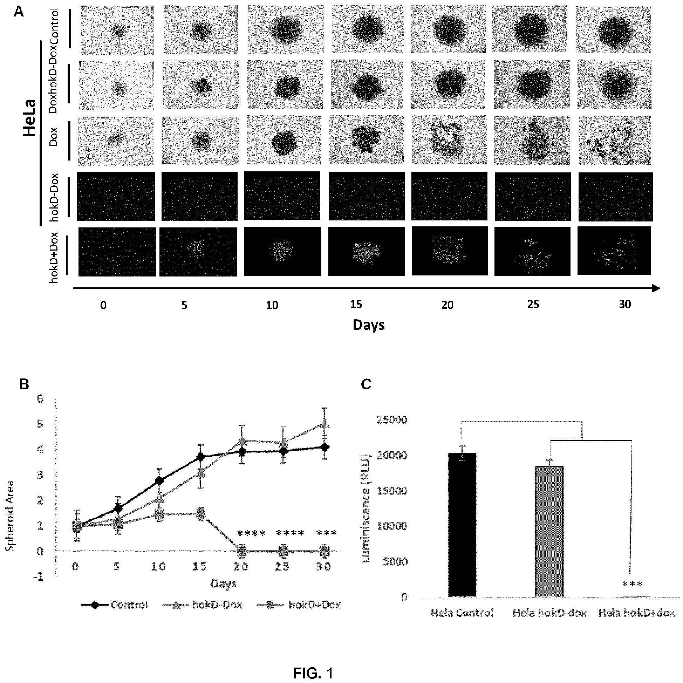

**Figure 1****. *hokD* gene expression induces complete destruction of spheroids of HeLa CSCs.** (A) HeLa *hokD* with Dox, without Dox and control HeLa were cultured for 30 days. Fluorescence and brightfield images were obtained using an EnSight system with well imaging technology. (B) Proliferation inhibition rate graph was obtained using imaged software, graph values were obtained by standardizing all days against day 0. (C) ATPlite level of Control, HeLa-hokD-Dox and HeLa-*hokD*+Dox assessed by ATPlite-3D assay kit. Values represent means ± SD (standard deviation) of triplicate cultures (*** p < 0.001 vs. control).

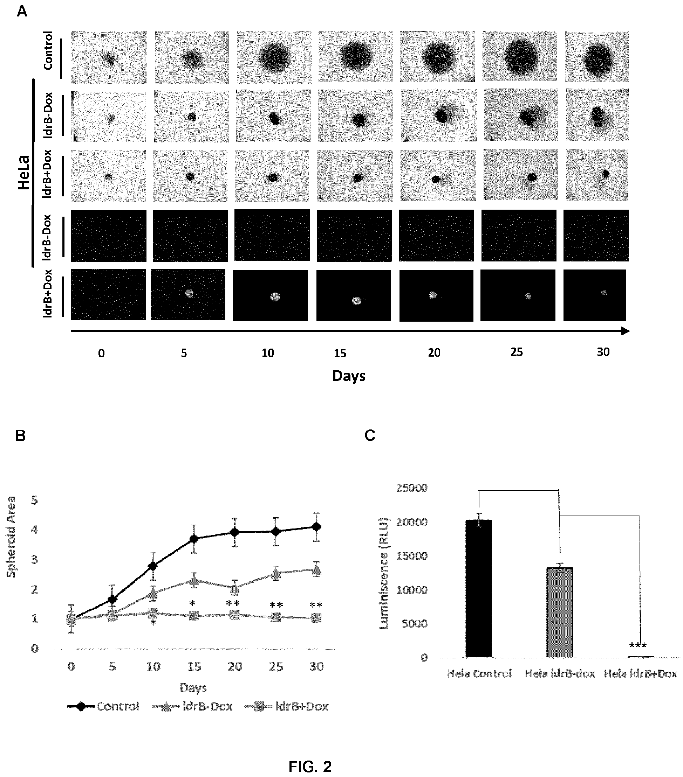

**Figure 2****. *IdrB* gene expression induces proliferation inhibition of HeLa spheroid CSCs.** (A) HeLa *IdrB* with Dox, without Dox and HeLa control were cultured for 30 days. Fluorescence and brightfield images were obtained using an EnSight system with well imaging technology. (B) Proliferation inhibition rate graph was obtained using imaged software; Graph values were obtained by standardizing all days against day 0. (C) Cell viability of Control, HeLa-*IdrB*-Dox and HeLa-IdrB+Dox assessed using the ATPlite-3D assay kit. Values represent means ± SD (standard deviation) of triplicate cultures (*** p < 0.001 vs. control).

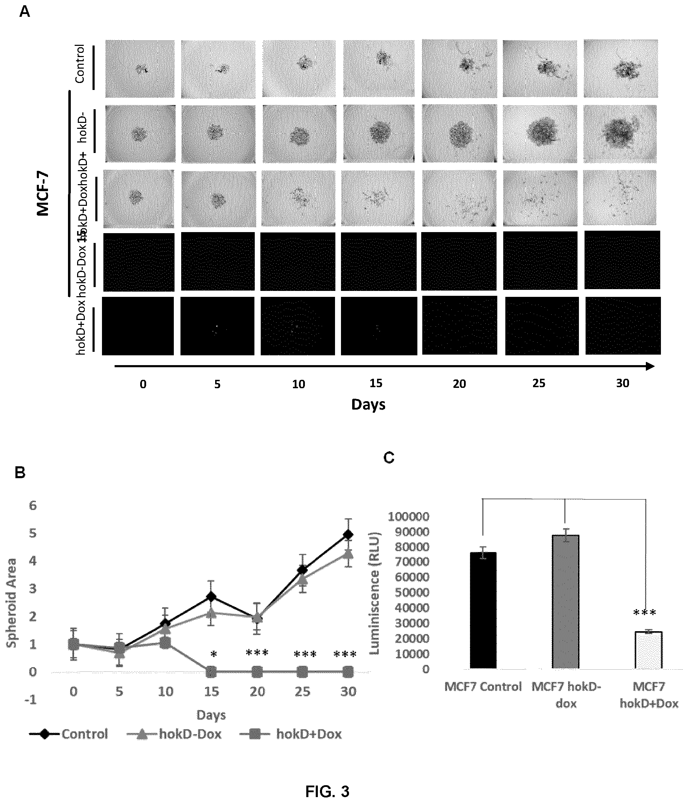

**Figure 3****. *hokD* gene expression induces inhibition of total destruction of MCF-7 spheroid CSCs.** (A) MCF-7 *hokD* with Dox, without Dox and control MCF-7 were cultured for 30. Fluorescence and brightfield images were obtained using an EnSight system with well imaging technology. (B) Inhibition proliferation rate graph was obtained using imaged software; Graph values were obtained by standardizing all days against day 0. (C) Cell viability of Control, MCF-7-*hokD*-Dox and MCF-7-*hokD*+Dox was assessed using ATPlite-3D assay kit. Values represent means ± SD (standard deviation) of triplicate cultures (*** p < 0.001 vs. control).

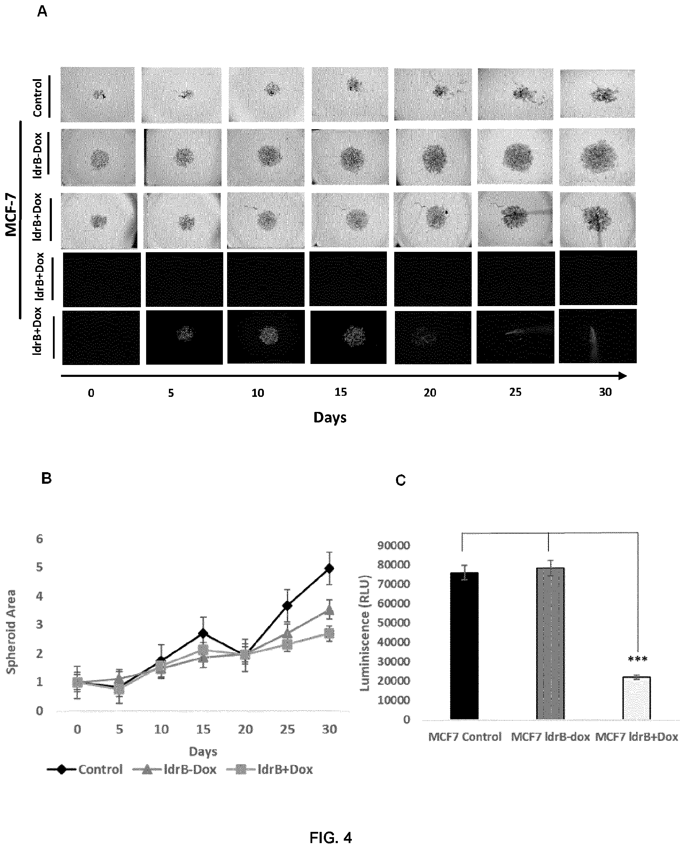

**Figure 4****. *IdrB* gene expression induces inhibition of MCF-7 spheroid proliferation.** (A) MCF-7 *IdrB* with Dox, without Dox and control MCF-7 were cultured for 30 days to determine the disintegration and proliferation rate. Fluorescence and brightfield images were obtained using an EnSight system with well imaging technology. (B) The inhibition proliferation rate graph was obtained using imaged software. Graph values were obtained by standardizing all days against day 0. (C) Cell viability of Control, MCF-7-/drB-Dox and MCF-7-/drB+Dox was determined by ATPlite assay. Values represent means ± SD (standard deviation) of triplicate cultures (*** p < 0.001 vs. control).

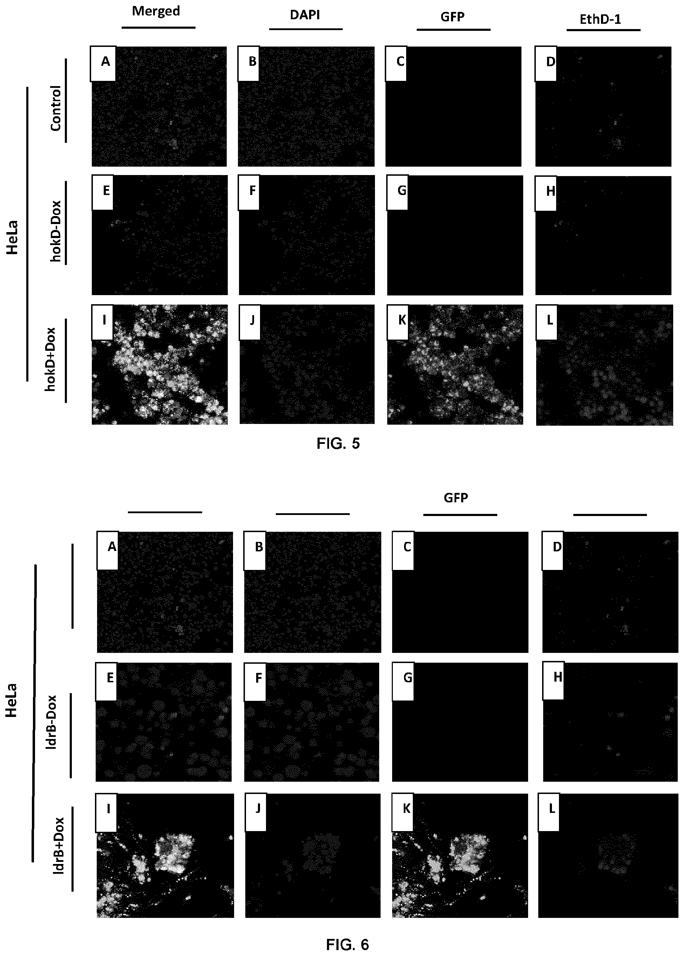

**Figure 5****. Confocal microscopy studies confirm that the *hokD* gene induces a loss of cell viability.** DAPI staining of Control and HeLa *hokD-Dox* (B, F) showed uniform nuclear staining and a large number of cells, whereas in the HeLa *hokD+Dox* cell line showed fewer cells and irregular nuclei staining (J). Cells stained with EthD-1 showed a loss of membrane integrity (L). Green fluorescence in GFP confirms correct induction of transfected cells (K).
**Figure 6****. Confocal microscopy studies confirm that the *IdrB* gene induces a loss of cell viability.** DAPI staining of Control and HeLa *IdrB*-Dox (B, F) showed uniform nuclear staining and a large number of cells, whereas in the HeLa *IdrB*+Dox cell line showed fewer cells and irregular nuclei staining (J). EthD-1 stained cells were observed showing a loss of membrane integrity (L). Green fluorescence in GFP confirms correct induction of transfected cells (K).

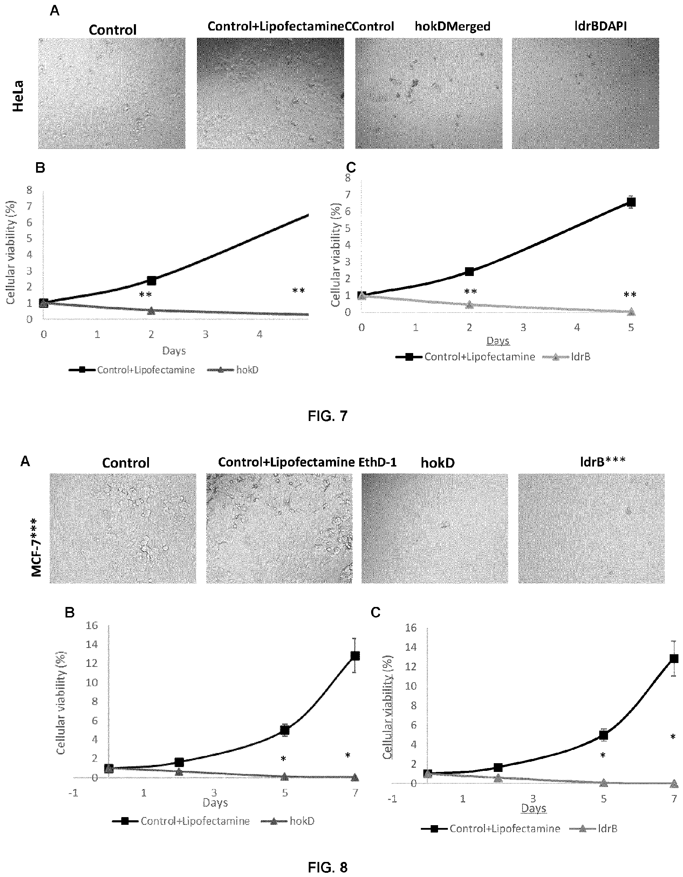

**Figure 7****. Gene expression of *hokD* and *IdrB* under the control of the survivin promoter induces inhibition of HeLa proliferation.** (A) HeLa, HeLa+lipofectamine, HeLa-hokD and HeLa-/drB were cultured for 5 days to determine the growth rate. (B) Cell viability of HeLa+lipofectamine and HeLa-*hokD* on different days (0, 2 and 5) of gene transfection. (C) Cell viability of HeLa+lipofectamine and HeLa-*IdrB* on different days (0, 2 and 5) of gene transfection. Values represent means ± SD (standard deviation) of triplicate cultures (* p < 0.05; ** p < 0.01; *** p < 0.001).
**Figure 8****. Gene expression of *hokD* and *IdrB* under the control of the survivin promoter induces inhibition of MCF-7 proliferation.** (A) MCF-7, MCF-7+lipofectamine, MCF-7-*hokD* and MCF-7-*IdrB* were cultured for 7 days to determine the growth rate. (B) Cell viability of MCF-7+lipofectamine and MCF-7-*hokD* on different days (0, 2, 5 and 7) of gene transfection. (C) Cell viability of MCF-7+lipofectamine and MCF-7-*hokD* on different days (0, 2, 5 and 7) of gene transfection. Values represent means ± SD (standard deviation) of triplicate cultures (* p < 0.05; ** p < 0.01; *** p < 0.001).

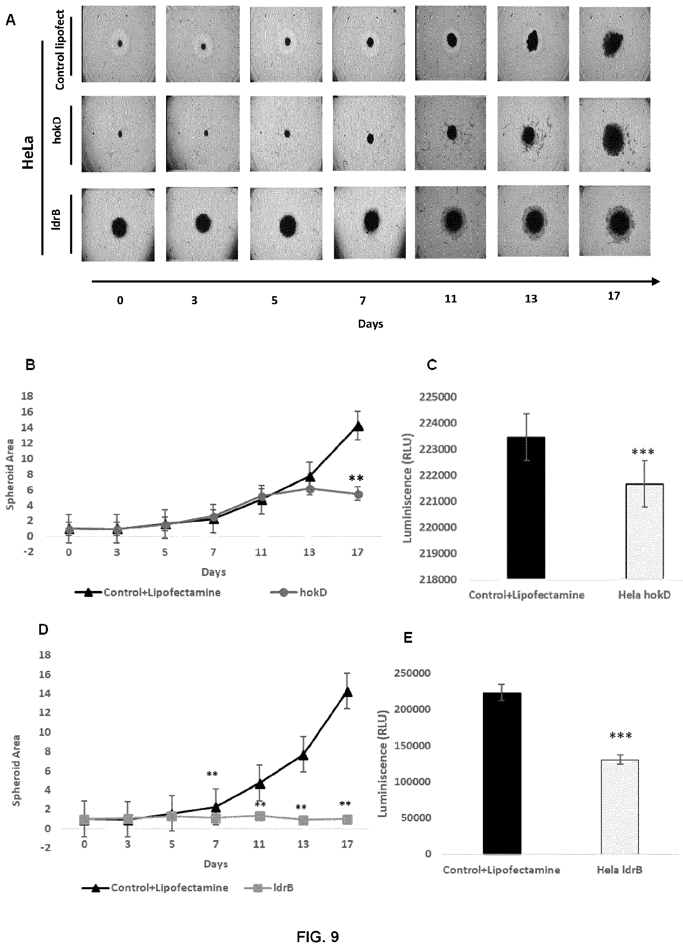

**Figure 9****. Gene expression of *hokD* and *IdrB* under the control of the survivin promoter induces proliferation inhibition in HeLa spheroid.** (A) Control, HeLa *hokD* and HeLa *IdrB* were cultured for 17 days. Brightfield images were obtained using an EnSight system with well imaging technology. (B, D) Inhibition proliferation rate graph was obtained using imaged software; Graph values were obtained by standardizing all days against day 0. (C, E) Cell viability of Control, HeLa *hokD* and HeLa *IdrB* assessed using the ATPlite-3D assay kit. Values represent means ± SD (standard deviation) of triplicate cultures (** p<0.01 vs control and *** p<0.001 vs control).

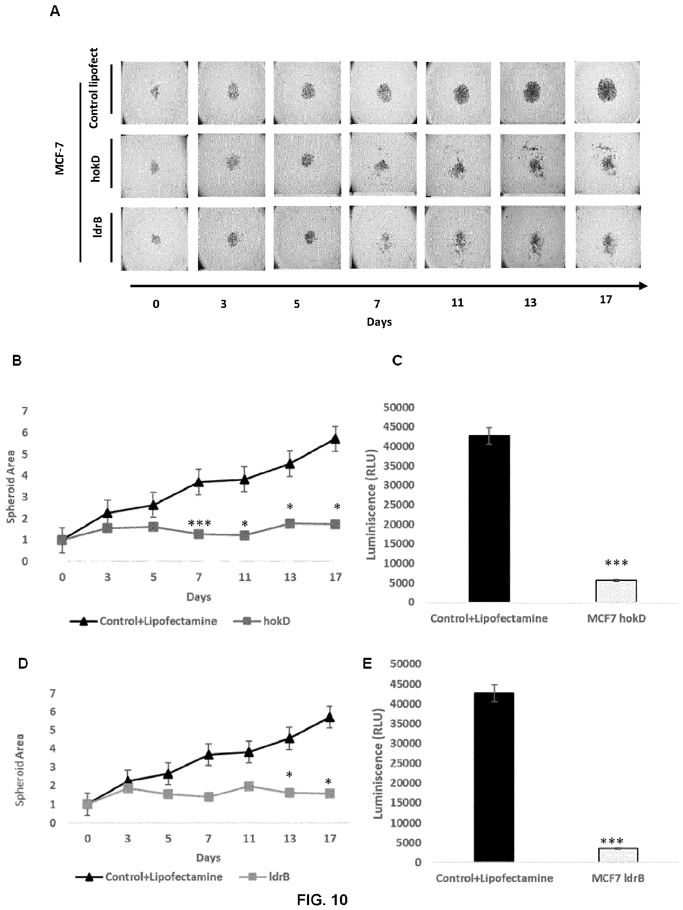

**Figure 10****. Gene expression of *hokD* and *IdrB* induces inhibition of proliferation in MCF-7 spheroid under the control of survivin promoter.** (A) Control, MCF-7 *hokD* and MCF-7 *IdrB* were cultured for 17 days to determine the disintegration and proliferation rate. Brightfield images were obtained using an EnSight system with well imaging technology. (B, D) Inhibition proliferation rate graph was obtained using imaged software; Graph values were obtained by standardizing all days against day 0. (C, E) ATPlite level of Control, MCF-7 *hokD* and MCF-7 *IdrB* assessed by ATPlite-3D assay kit. Values represent means ± SD (standard deviation) of triplicate cultures (* p < 0.05 vs. control and *** p < 0.001 vs. control).

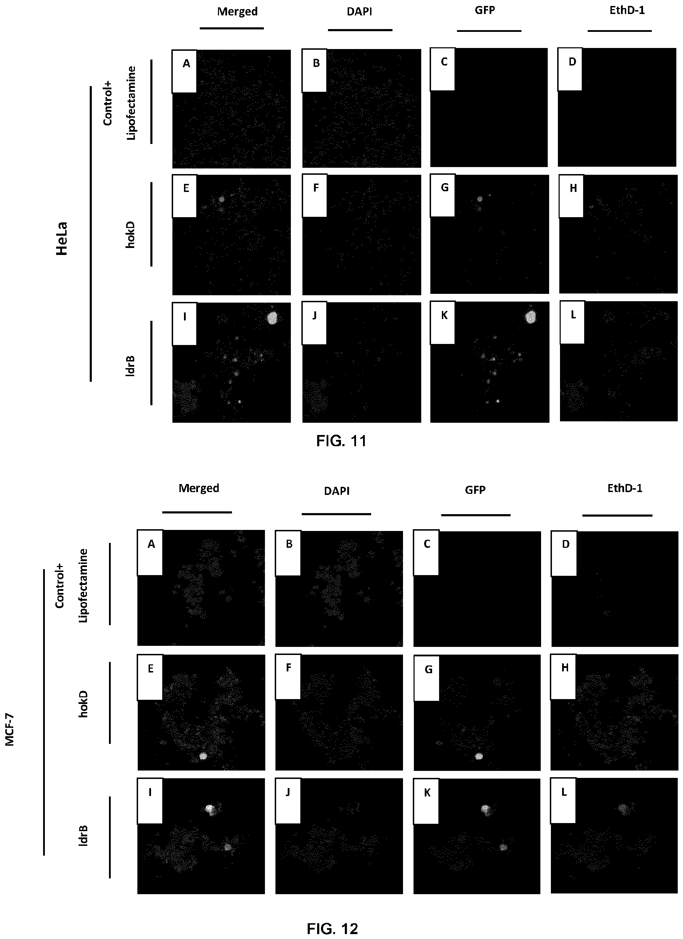

**Figure 11****. Confocal microscopy studies confirm significant differences in growth patterns and inhibition of proliferation with the *hokD* and *IdrB* gene in HeLa cells.** DAPI staining of the Control (B) showed uniform nuclear staining and a large number of cells, whereas in the HeLa *hokD* cell line showed fewer cells and irregular nuclei staining (J). EthD-1 stained cells were observed showing a loss of membrane integrity (L). Green fluorescence in GFP confirms correct induction of transfected cells (K).
**Figure 12****. Confocal microscopy studies confirm significant differences in growth patterns and inhibition of proliferation with *hokD* and *IdrB* gene in MCF-7 cells.** DAPI staining of the Control (B) showed uniform nuclear staining and a large number of cells, whereas in the HeLa cell line *hokD* showed fewer cells and irregular nuclei staining (J). EthD-1 stained cells were observed showing loss of membrane integrity (L).

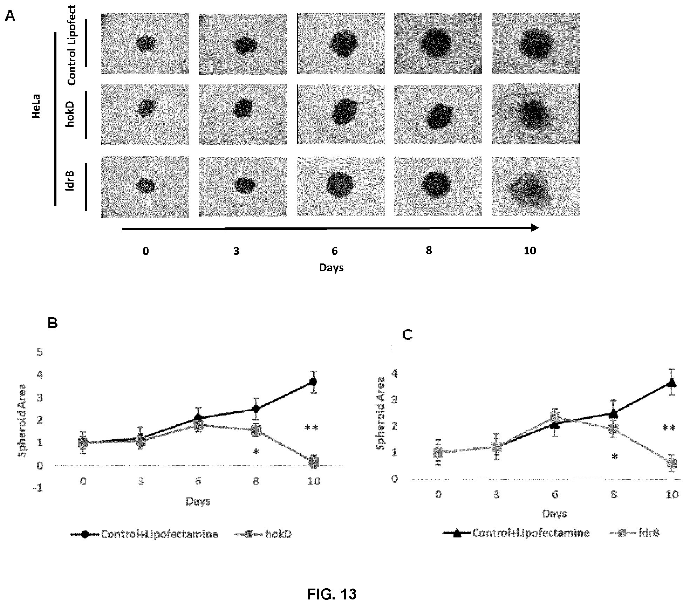

**Figure 13****. Gene expression of *hokD* and *IdrB* under the control of the survivin promoter induces proliferation inhibition in HeLa spheroid CSCs.** (A) Control, HeLa *hokD* and HeLa *IdrB* were cultured for 10 days to determine the disintegration and proliferation rate. Brightfield images were obtained using an EnSight system with well imaging technology. (B, C) Inhibition proliferation rate graph was obtained using imaged software; Graph values were obtained by standardizing all days against day 0. Values represent means ± SD of triplicate cultures (* p < 0.05 vs. control and *** p < 0.001 vs. control).

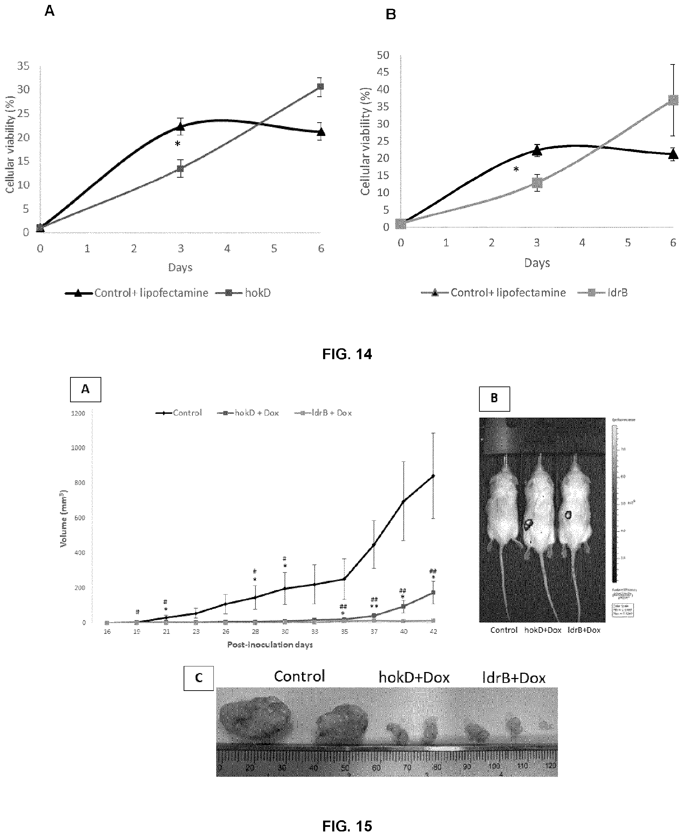

**Figure 14****. Gene expression of *hokD* and *IdrB* under the control of the survivin promoter induces follicle proliferation.** (A) Cell viability of follicle+lipofectamine and follicle-hokD on different days (0, 3 and 6) of gene transfection. (B) Cell viability of follicle+lipofectamine and follicle-IdrB on different days (0, 3 and 6) of gene transfection. Values represent means ± SD (standard deviation) of triplicate cultures (* p < 0.05; ** p < 0.01; *** p < 0.001).
**Figure 15****. Volume (mm3) of HeLa CSCs tumors transfected with *hokD* and *IdrB* under the control of the TRE3G-Dox promoter.** (A) Volume (mm3) of HeLa CSCs tumors transfected with *hokD* and *IdrB* under the control of the TRE3G-Dox promoter compared to control. (B) *In vivo* fluorescence images of tumors in mice. (C) Excised tumors. Values represent means ± SD (standard deviation) of triplicate cultures (*hokD* significance: * p < 0.05; ** p < 0.01; *** p < 0.001; *IdrB* significance: # p < 0.05; ## p < 0.01; ### p < 0.001).

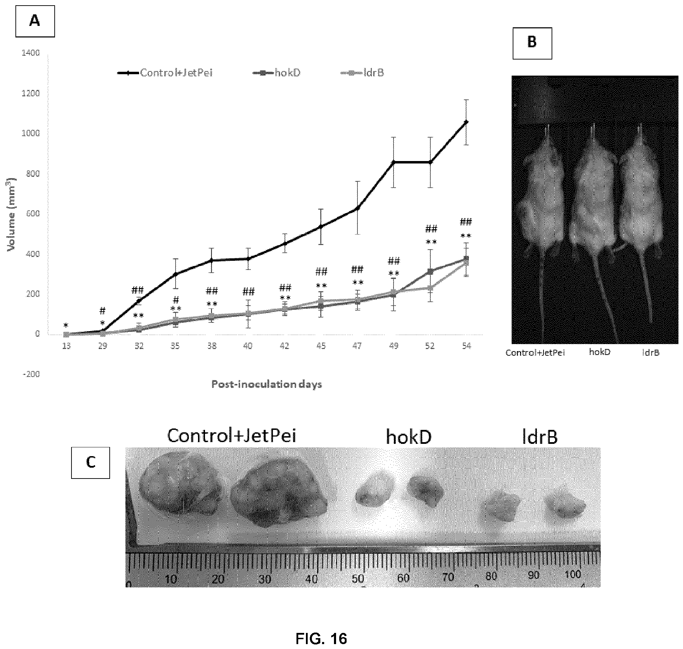

**Figure 16****. Volume (mm3) of HeLa CSC tumors transfected with *hokD* and *IdrB* under the control of the survivin promoter.** (A) Volume (mm3) of HeLa CSC tumors transfected with *hokD* and *IdrB* under the control of the survivin promoter compared to control. (B) *In vivo* images of tumor size in mice. (C) Excised tumors. Values represent means ± SD (standard deviation) of triplicate cultures (*hokD* significance: * p < 0.05; ** p < 0.01; *** p < 0.001; *IdrB* significance: # p < 0.05; ## p < 0.01; ### p < 0.001).

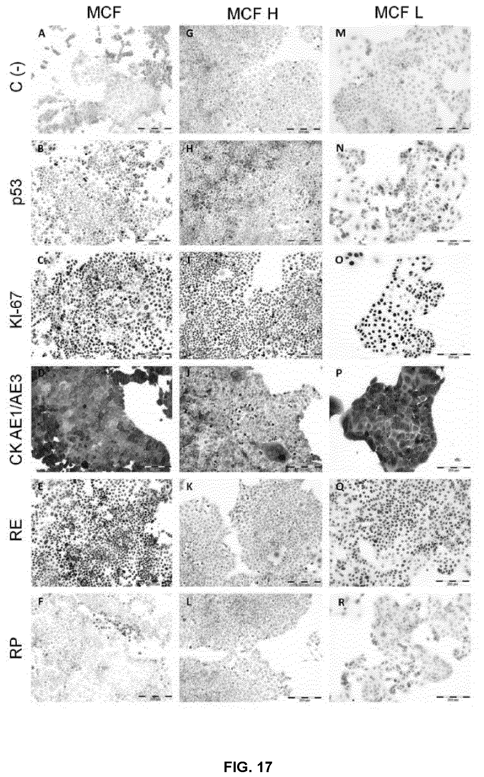

Figure 17. Immunocytochemical analysis of p53 protein, ki-67 antigen, CKAE1/AE3, estrogen receptor (ER) and progesterone receptor (PR) in control MCF-7, MCF-7 *hokD* and MCF-7 *IdrB.*

### DETAILED DESCRIPTION OF THE INVENTION

### Materials and methods

Cell lines and cultures; Human cervical cancer cell line HeLa was obtained from ATCC. Human breast cancer cell line MCF-7 was obtained from Biobanco del Sistema Público de Salud de Andalucia (BBSSPA), Granada, Spain. Human Hair Follicle Stem Cells were obtained from Quimigen S.L. Plasmid constructs; the genes chosen to be expressed in the target cells were *hokD* and *IdrB.* Due to the need to specifically express specific genes in the target cells, promoters are used to help achieve this goal. First, the Tet-On^{®} 3G inducible expression system, which contains the pTRE3G-mCherry and pCMV-Tet3G vectors purchased from Clontech, is used. It is a doxycycline-inducible promoter that allows the expression of genes under its control only in the case that the cell has doxycycline. Thus, we could express the suicide genes whenever necessary only by administering doxycycline. On the other hand, we use the survivin gene-based expression system, which is overexpressed in cancer cells. [7] When this gene is translated, its function as an apoptosis inhibitor is crucial in cancer cell growth. This promoter is considered a tissue-specific promoter and will facilitate the expression of suicide genes specifically in target genes.

### Two-dimensional cell proliferation assay

Cell lines were seeded in 96-well plates. Cells from 2D assays were cultured in Dulbecco's modified Eagle's medium (DMEM) (Sigma-Aldrich) supplemented with 10% fetal bovine serum (FBS) (Sigma-Aldrich) and 1% penicillin/streptomycin (P/S) (Sigma-Aldrich) cells were incubated at 37°C under air containing 5% CO₂. Transfections were performed transiently using the Lipofectamine^{™} 3000 Reagent protocol (Invitrogen, ThermoFisher Scientific) every two days for cells transfected with survivin promoter. In addition, two different dilutions of lipofectamine (1/33 and 1/22) were tested. Cell viability measurements were performed with the alamarBlue^{™} Cell Viability Reagent protocol, prior to each new transfection.

### Three-dimensional cell growth assay

As for 3D cultures, differentiated cell spheroids and CSCs were made. Differentiated cell spheroids were cultured in Dulbecco's modified Eagle's medium (DMEM) (Sigma-Aldrich) supplemented with 10% fetal bovine serum (FBS) (Sigma-Aldrich) and 1% penicillin/streptomycin (P/S) (Sigma-Aldrich), without phenol red. CSCs were prepared in cone-based 96-well plates with conventional sphere medium (DMEM:F12) (Sigma-Aldrich), 1 % penicillin/streptomycin (P/S) (Sigma-Aldrich), B27 (Gibco Life Technologies, USA), 10 µg/mL ITS (Gibco Life Technologies, USA.), 1 µg/mL hydrocortisone (Sigma-Aldrich), 4 ng/mL heparin (Sigma-Aldrich), 20 ng/mL epidermal growth factor (EGF) (Sigma-Aldrich), 10 ng/mL fibroblast growth factor (FGF) (Sigma-Aldrich), 10 ng/mL hepatocyte growth factor (HGF) (Sigma-Aldrich), 10 ng/mL interleukin-6 (IL-6) (Sigma-Aldrich), without phenol red). Plates were incubated at 37°C under air containing 5% CO₂.

Cell Carrier Spheroid ULA 96-well Microplates (PerkinElmer) according to manufacturer's instructions. Clear 96-well polystyrene microplates coated with ultra-low attachment (ULA) surface and round bottom for 3D culture of mammalian cells. This ULA coating on round-bottom wells facilitates the formation of round spheroids after incubating the seeded cells. Spheroids were imaged using a 4x objective on the imaging module of an EnSight TM plate reader (PerkinElmer). The images obtained were analyzed using imaged software.

Transfections were performed in a stable or integrative way using the Xfect^{™} Transfection Reagent Protocol-At-A-Glancein (Clontech Laboratories, Inc. A Takara Bio Company) the case of cells transfected with the TRE3G-Dox promoter, which was induced with 2 µg/ml doxycycline three times per week; and, transiently using the Lipofectamine^{™} 3000 Reagent Protocol (Invitrogen, ThermoFisher Scientific) every two days in the case of cells transfected with survivin promoter. In addition, two different dilutions of lipofectamine (1/33 and 1/22) were tested. In three-dimensional cultures, measurements were performed in imaged software.

Confocal imaging; DAPI and Ethidium homodimer-1 (EthD-1) were used for 3D assays endpoint to confirm well imaging results. At the end of the 3D cell growth assay, spheroids were stained with DAPI (1 µg/ml) and EthD-1 (2 µM) in PBS at room temperature for 20 min for EthD-1 and 5 min for DAPI. Images were then taken by confocal microscopy using a Leica SP2 confocal microscope (Telstar Instrumat, Terrassa, Barcelona, Spain).

ATPlite-based luminescence assays; the ATPlite 3D kit (PerkinElmer, Waltham, MA, USA) was used. Using this kit, ATPlite content can be measured at the endpoint measurements of three-dimensional cultures. According to the protocol, a reagent containing luciferase and luciferin was added to differentiated cell spheroids, which were lysed at the end. Using the EnSight system, luminescence was measured.

### In vivo antitumor xenograft studies

*In vivo* studies were performed with a cervical cancer model.

TRE3G-Dox promoter; first, the therapeutic efficacy of suicide gene therapy with *hokD* and *IdrB* under the control of the Tet-On system was evaluated using heterotopic tumor xenografts established with HeLa, HeLa-hokD and HeLa-/drB cell lines. A total of 6000 CSCs per mouse were injected subcutaneously into BALB/c mice. After tumor palpation, 100 mg/kg doxycycline was administered 3 times per week by intraperitoneal injection. Controls used for comparison were mice with HeLa tumor but without doxycycline administration.

Survivin promoter; for evaluation of the therapeutic efficacy of *hokD-* and *IdrB-*mediated suicide gene therapy under the control of the survivin promoter, a cancer-specific system was initiated by injecting 6000 CSCs per mouse to generate heterotopic tumor xenografts established with HeLa, HeLa-*hokD* and HeLa-/drB cell lines. CSCs were injected subcutaneously into BALB/c mice. After tumor palpation, the *in vivo*-jetPEI^{®} system of the plasmid with the *hokD* and *IdrB* genes was administered separately. The system consists of a linear polyethylenimine, which facilitates the efficient delivery of nucleic acids such as DNA, hcRNA, siRNA, miRNA, among others, with different delivery possibilities and different tissues where make it. Treated mice were administered 3 times per week intratumorally with 20 µg of DNA in 100 µl of 5% sucrose solution and a N/P=6 ratio of jetPEI. In the case of control mice, the same solution was administered without DNA.

### Immunoflorescence

Prior to immunocytochemical analysis, 25,000 cells from each line were subcut into a plate. Every 48 hours, doxycycline was added to the cell culture in all cell lines except control cells. After 6 days of gene induction, the cells were fixed. Cells were washed twice with fetal bovine serum (PBS) and then fixed with paraformaldehyde (PFA) for 20 minutes and washed twice more. The samples were initially washed (3 washes of 5 minutes each) with wash solution to remove fixative residues. They were then incubated for 15 minutes in permeabilization solution (Triton-X100 1%). Once the samples were permeabilized, they were washed with washing solution (2 x 5 min) and the staining process was started, consisting of the following steps: peroxidase inhibitor (5 min), washing with washing solution (2 x 5 min), primary antibody (20 min), washing with washing buffer (2 x 5 min), secondary antibody (20 min), diaminobenzidine (DAB) development solution (10 min) and counterstaining with hematoxylin (15 min). The whole process was carried out at room temperature. A negative control was performed on each plate, replacing the primary antibody with PBS.

### Statistical analysis

All data presented in this paper are expressed as the mean ± SD (standard deviation) of three replicates and compared with Student's t-tests. Values of p < 0.05 (*, #), values of p < 0.01 (**, ##) and values of p < 0.001 (***, ###) were considered statistically significant in all cases.

### Gene expression of hokD and IdrB promotes significant inhibition of proliferation in HeLa and MCF-7 cancer stem cell spheroids.

### Effect of hokD and IdrB expressed under the control of the TRE3G promoter on cell viability.

To study the effect of the *hokD* and *IdrB* gene in HeLa and MCF-7 cells, a 3rd generation Tet-On 3G inducible expression system with green fluorescent protein ("GFP") and m-Cherry was used. This generation is the most powerful, versatile and widely cited inducible mammalian expression system available and allows simultaneous expression of the *hokD* or *IdrB* gene and a green (HeLa) or red (MCF-7) fluorescence marker under the control of a TRE3G promoter (PTRE3G) that binds to the Tet-On 3G transactivator protein in the presence of doxycycline. Cells were transfected with *hokD* and *IdrB* separately and then CSCs were isolated and characterized. After 48 h of induction with Doxycycline (Dox) HeLa and MCF-7 CSCs spheroid showed expression of *hokD* and *IdrB* gene through GFP and m-Cherry fluorophore respectively (**Figure 1****,** **2****,** **3****,** **4**). The results demonstrate that both genes induce inhibition of proliferation and significant differences in growth patterns such as solidity, round and area rate compared to control (**Table 1, 2 and** **Figure 1B** **and** **2B**).

**HeLa;** in HeLa expressing *hokD* gene, we observe that cells undergo 52.8% inhibition of proliferation at 15 days reaching total disintegration after 30 days of treatment compared to the control without Dox (**Figure 1A****, B**). For HeLa expressing the *IdrB* gene, we observed that the spheroid undergoes growth inhibition but not total disintegration. Proliferation inhibition reached 52.28% and 61.64% after 15 and 30 days of treatment on relation to control without Dox (**Figure 2A****, B**). This inhibition was in concordance with the decrease in ATPlite level observed, compared to the control, at the end of the experiments with the ATP lite assay kit (Adenosine triphosphate (ATP) monitoring system) for 99.45 % of the *hokD* gene (**Figure 1C**) and 99.16 % of the *IdrB* gene (**Figure 2C**).

**Table 1. Roundness and solidity values of control HeLa CSC and transfected with hokD and IdrB obtained as mean of endpoint measurements.**

| | HeLa CSCs | | | | |
|---|---|---|---|---|---|
| | Control | *hokD*-Dox | *hokD*+Dox | *IdrB*-Dox | *IdrB*+Dox |
| Roundness | 1.06 | 0.972 | 0 | 1.3 | 0.964 |
| Solidity | 1 | 1.04 | 0.556 | 1.02 | 1.034 |

Roundness and solidity values go from 0 to 1, being more round and solid when the value is nearest to 1. The values in the table were obtained by standardizing all days versus 0 day.

**MCF-7;** MCF-7 CSCs spheroid expressing the *hokD* gene undergoes 31.54% inhibition of CSCs spheres proliferation after 10 days of treatment and total disintegration from day 15 compared to the control transfected with *hokD* without Dox induction (Figure 3A, B). These results are in concordance with those obtained through the survival study using the ATPlite-3D assay kit obtaining 71.7% inhibition of cell viability at the end of the experiment (**Figure 3C**). MCF-7 expressing the *IdrB* gene showed a slight decrease of CSCs sphere area reaching only 13.7% and 23.46% after 10 and 30 days of treatment compared to the control transfected with the *IdrB* gene without Dox induction (**Figure 4A****, B**). Furthermore, as in HeLa expressing *IdrB* gene, total disintegration of the spheroid was not observed. However, cell viability determined by ATPlite assay at the end of the experiment showed a clear decrease in viability reaching 72.07% (**Figure 4C**). This indicates that although the sphere of CSCs has not disintegrated, most of the cells are dead.

**Table 2. Roundness and solidity values of control MCF-7 CSC and transfected with hokD and IdrB obtained as mean of endpoint measurements.**

| | MCF-7 CSCs | | | | |
|---|---|---|---|---|---|
| | Control | *hokD*-Dox | *hokD*+Dox | *IdrB-*Dox | *IdrB*+Dox |
| Roundness | 1.00 | 0.983 | 0 | 1.00 | 0.830 |
| Solidity | 1.01 | 1.05 | 0.449 | 0.994 | 1.02 |

Roundness and solidity values go from 0 to 1, being more round and solid when the value is nearest to 1. The values in the table were obtained by standardizing all days versus 0 days.

These results suggest that although the *hokD* and *IdrB* genes affect cell proliferation, their mechanism of action is different. In this sense, in both the cervical and breast cancer lines, the *hokD* **gene causes the total disintegration of the treated spheres while *IdrB* blocks cell proliferation,** keeping the size of the sphere the same throughout the treatment.

### Confocal assay

Confocal microscopy studies were performed to demonstrate significant differences in growth patterns and inhibition of proliferation. DAPI staining of Control, HeLa *hokD-Dox* and HeLa-*Idr*B-Dox (**Figure 5B,** **F**) (**Figure 6B, F**) showed uniform nuclear staining and high cell number. In contrast, EthD-1 staining showed only those cells that exhibit a loss of membrane integrity, as can be observed in HeLa *hokD*+Dox and HeLa *IdrB*+Dox (**Figure 5L and 6L**). GFP represents the expression of the fluorescence marker under the control of the TRE3G promoter (PTRE3G) only expressed in cells transfected with *hokD* and *IdrB* induced by doxycycline (**Figure 5K and 6K**) and absent in cells transfected without Dox and control cells (**Figure 5C, G**) (**Figure 6C,** **G**). Merged images showed cells in which DAPI and EthD-1 staining matched, indicating cells that present nuclei but have lost membrane integrity. **In the case of transfected and induced HeLa *hokD*+Dox cells, it can be seen how all cells were dead. However, HeLa *IdrB+Dox* demonstrated the presence of dead cells combined with live cells.** In the case of HeLa *hokD-Dox,* HeLa *IdrB-*Dox and Control cells, we found a large amount of nuclei stained with DAPI but without EthD-1 overlay, indicating cell viability.

### Effect of hokD and IdrB expressed under the control of the TRE3G promoter on tumor biomarkers.

Immunocytochemical analysis demonstrated modifications in the tumor biomarker panel of MCF-7 cells transfected with the *hokD* and *IdrB* gene compared to control cells. The percentages of stained cells and signal intensity are shown in **Table 3.**

**Table 3: Immunocytochemical Analysis**

| | **P53** | **Ki-67** | **CKAE1/AE3** | **ER** | **PR** |
|---|---|---|---|---|---|
| **MCF-7 Control** | 2, ++/+++ | 4, +++ | 4, +++ | 4, +++ | 1, ++/+++ |
| **MCF-7 H** | 1, ++/+++ | 4, ++ | 4, ++/+++ | 0, - | 0, - |
| **MCF-7 L** | 1, ++/+++ | 4, ++ | 4, +++ | 4, +++ | 0, ++/+++ |

The results obtained indicate once again that *hokD* and *IdrB* genes do not have the same mechanism of action. Thus, breast cancer cells treated with *hokD* showed a loss of ER and PR hormone receptor expression and a decrease in p53 and CKAE1/AE3 proteins. In MCF7 expressing *IdrB* gene showed a slight decrease in ER and PR expression and p53 protein but ki-67 and CKAE1/CKAE3 tigers had no difference with respect to control cells. However, both genes showed a moderate ki-67 signal (**Table 3 and** **Figure 17**).

### Effect of hokD and IdrB expressed under the control of the survivin promoter.

To direct the expression of *IdrB* and *hokD* genes to tumor tissue in general, and CSCs in particular, a plasmid has been constructed whose expression is dependent on the survivin promoter. Survivin is known to be overexpressed in tumor cells compared to healthy tissue. Therefore, HeLa and MCF-7 cells were transiently transfected with the *hokD* and *IdrB* gene separately for 2D and 3D cultures. Lipofectamine was used as a vehicle for transfection using lipid nanoparticles to provide superior transfection performance with improved application outcomes and reproducible results. In 2D cultures, significant differences in growth patterns were found. After 48 h of transfection of HeLa cells, the *hokD* gene was able to induce 76.89 % and 95.78 % inhibition of proliferation at 2 and 5 days, respectively. Similarly, the *IdrB* gene was able to induce 52.68 % and 99.36 % proliferation inhibition at day 2 and 5, respectively (**Figure 7**). The same trend was observed in MCF-7, the results showed 61.46 %, 98.17 % and 99.63 % inhibition of proliferation on days 2, 5 and 7, respectively, after *hokD* gene induction; and 63.87 %, 94.48 % and 99.85 % on day 2, 5 and 7 respectively after *IdrB* gene expression (**Figure 8**).

To verify the effect of the *hokD* and *IdrB* gene under the control of the survivin promoter in HeLa and MCF-7 spheroid differentiated cells and CSCs, 3D proliferation assays were performed. As shown in **Figure 9****,** significant differences were found in proliferation and growth patterns, such as solidity, round and area rate. In the case of area, the values seem to decrease in the case of *hokD* while it remains lower than the control in *IdrB.* In roundness and solidity the parameters are similar to the control (**Table 4**) (**Figure 9B**).

For HeLa with *hokD* gene, cells were observed to suffer disintegration around the spheroid and inhibition of proliferation after 13 days (19.66%). This inhibition reached 61.29% after 17 days of transfection compared to the control (**Figure 9A****, B**). For HeLa with *IdrB* gene there was a high disintegration around the sphere and a high percentage of proliferation inhibition. Cells underwent growth inhibition after 13 days, the inhibition showing values of 87.69%. This inhibition increased after 17 days reaching 92.82% in relation to the control (**Figure 9A****, D**). This inhibition was in concordance with the decrease in ATPlite level observed at the end of the experiments with the ATP lite assay kit of 0.8% for the *hokD* gene (**Figure 9C**) and of 41.44%for the *IdrB* gene (**Figure 9E**).

The proliferation study showed significant growth inhibition of MCF-7 spheroids compared to control cells. After 13 days of *hokD* and *IdrB* gene induction, the size of the spheroids was reduced compared to the control. The results obtained showed that after 13 days of gene expression there was an inhibition of proliferation of 36.87% and 64.14% for *hokD* and *IdrB.* This inhibition increased, reaching 66.08 % and 74.65 % after 17 days of treatment for *hokD* and *IdrB* respectively (**Figure 10** **A, B, D**). Moreover, this inhibition was in agreement with the decrease in ATPlite level observed at the end of the experiments with the ATP lite assay kit for 85.75 % of the *hokD* gene (**Figure 10C**) and 91.59 % of the *IdrB* gene (**Figure 10E**).

**Table 4: Roundness and solidity of HeLa and MCF-7 spheroid, transfected with hokD and IdrB under the control of the survivin promoter, obtained as an average of endpoint measurements.**

| | Differentiated HeLa cell spheroids | | | MCF-7 differentiated cell spheroids | | |
|---|---|---|---|---|---|---|
| | Control + Lipofectamine | *hokD* | *IdrB* | Control + Lipofectamine | *hokD* | *IdrB* |
| Roundness | 0.982 | 0.748 | 0.964 | 1.31 | 0.916 | 0.834 |
| Solidity | 1.025 | 0.995 | 1.02 | 1.11 | 0.816 | 0.705 |

Roundness and solidity values range from 0 to 1, being more round and solid when the value is nearest to 1. The values in the table were obtained by standardizing all days versus 0 days.

### Confocal assay

Confocal microscopy studies were performed to test for significant differences in growth patterns and inhibition of proliferation. DAPI staining of Control, HeLa and MCF-7 *hokD* and HeLa and MCF-7 *IdrB* **(****Figure 11B, F,** **J) (Figure 12B,** **F,** **J)** showed uniform nuclear staining and high cell number. In contrast, EthD-1 staining showed only those cells exhibiting loss of membrane integrity, as can be observed in HeLa and MCF-7 *hokD* and HeLa and MCF-7 *IdrB* **(****Figure 11L, H) (****Figure 12L,** **H).** GFP represents the expression of the fluorescence marker under the control of the survivin promoter, expressed only in cells transfected with *hokD* and *IdrB* **(****Figure 11K)** (Figure **12K).** Merged showed cells where DAPI and EthD-1 staining coincide are cells that present nuclei but have lost membrane integrity and do not express fluorescence, this is the case of HeLa and MCF-7 *hokD,* HeLa and MCF-7 *IdrB* transfected cells. These cells are considered to be dead and a confirmation of the results of inhibition of proliferation. In the case of control HeLa cells, we found a large number of nuclei stained with DAPI but no EthD-1 overlay, indicating cell viability.

### Effect of hokD and IdrB gene on cancer stem cells: 3D proliferation assay in HeLa cell line.

The proliferation study shows significant growth inhibition of HeLa spheroids compared to control cells. After 8 days of *hokD* and *IdrB* gene expression, spheroid size was 37.87 % for *hokD* and 24.04 % for *IdrB.* This inhibition increased to 95.5% and 83.99% after 10 days of treatment for *hokD* and *IdrB* respectively observing complete sphere disintegration for both genes (**Figure 13A****, B, D**). The solidity and roundness values reinforce the data presented, due to their loss of shape and showing that the sphere disintegrated inside (**Table 5**).

**Table 5. Roundness and solidity values of HeLa spheroids CSCs transfected with hokD and IdrB under the control of the survivin promoter, obtained as an average of endpoint measurements.**

| | HeLa CSCs | | |
|---|---|---|---|
| | Control+Lipofectamine | *hokD* | *IdrB* |
| Roundness | 0.88 | 0.33 | 0.45 |
| Solidity | 1.00 | 0.39 | 0.50 |

Roundness and solidity values range from 0 to 1, being more round and solid when the value is nearest to 1. The values in the table were obtained by standardizing all days versus 0 day.

### Gene expression of hokD and IdrB under the control of the survivin promoter does not affect follicular cell proliferation.

In order to verify the tissue-specific action of the construct and its safety in healthy cells, non-tumor follicular stem cells were transfected with the same protocol. The main objective of these transfections was to determine the survivin promoter cancer cells specificity, in order to corroborate the safety of the treatment, due to its scarce expression in non-tumor cells. The results obtained show that non-tumor cells grow normally, which confirms the safety of the use of the survivin promoter in suicide gene therapy, due to the tumor-specific action of the promoter. The percentage of proliferation shows a growth of 1.5 times more than the control, with 143.76% and 174.12% cell growth in cells transfected with *hokD* and *IdrB* respectively after 6 days of treatment (**Figure 14A****, B**).

### In vivo antitumor xenograft studies

To corroborate the results obtained in two- and three-dimensional cultures and to evaluate the therapeutic efficacy of suicide gene therapy, *in vivo* studies were performed in mice. These experiments were performed following the recommendations of the ethics committee; achieving a reduction in the number of animals and avoiding unnecessary suffering.

*In vivo* effect of *hokD* and *IdrB* under the control of the TRE3G-Dox promoter.

The results obtained in this *in vivo* experiment demonstrated significant inhibition of tumor proliferation in HeLa CSCs expressing *hokD* and *IdrB* under the control of the TRE3G-Dox promoter and activated with doxycycline after 42 days of treatment. We observed no significant differences in the growth of HeLa and HeLa-Dox tumors. The proliferation of HeLa *hokD* and HeLa *IdrB* tumors was significantly inhibited after 35 days with 91.6 % and 95.7 % respectively. This antitumor effect was maintained until the end of the experiment, maintaining an average tumor volume about 80% lower compared to the control group for *hokD* gene and about 98% for *IdrB* gene **(****Figure 15A****).** *In vivo* imaging of resected tumors and tumor-bearing mice confirmed *hokD* and *IdrB* gene expression in HeLa *hokD-Dox* and HeLa *IdrB*-Dox tumor (**Figure 15B****, C**). The treated mice showed no weight loss or other unusual behavior, no detectable toxicity, and no metastasis to the liver, lungs, heart, or kidneys. These events point to the effectiveness of the suicide genes without any systemic damage.

### In vivo effect of hokD and IdrB under the control of the survivin promoter.

The results obtained in this *in vivo* experiment demonstrated significant inhibition of tumor proliferation in HeLa CSCs expressing *hokD* and *IdrB* under the control of the survivin promoter after 54 days of treatment. The proliferation of HeLa *hokD* and HeLa *IdrB* tumors was significantly inhibited after 32 days with 76.21 % and 79.3 %, respectively. This antitumor effect was maintained until the end of the experiment, maintaining an average tumor volume about 66% lower compared to the control group for the *hokD* gene and about 67% for the *IdrB* gene **(****Figure 16A****).** *In vivo* imaging of resected tumors and tumor-bearing mice confirmed *hokD* and *IdrB* gene expression in HeLa *hokD-Dox* and HeLa *IdrB-Dox* tumor (**Figure 16B****, C**). The treated mice showed no weight loss or other unusual behavior, no detectable toxicity, and no metastasis to the liver, lungs, heart, or kidneys. These events point to the effectiveness of the suicide genes without any systemic damage.

As expected, the efficacy of the TRE3G-Dox promoter is much more potent than that of the survivin promoter. However, the mice had not shown any damage or metastasis, which means that the survivin promoter could be used as a tissue-specific promoter against breast and cervical cancer safely.

In the present report, *hokD* and *IdrB* gene expression was under the control of TRE3G-Dox as well as survivin. The use of induced promoter TRE3G controlled by doxycycline, a very potent promoter, also supports the efficacy of suicide gene expression in our cell lines. Only with the administration of this antibiotic, the signaling cascade will be triggered due to a doxycycline-binding transactivator protein [21]. In case doxycycline administration was suppressed, the expression of genes controlled by this promoter would remain silenced. The Tet-On system is widely used in this type of suicide gene therapy research.

The tissue-specific promoter chosen in this case is the survivin promoter, whose activity increases during the cell cycle. This choice is due to the National Cancer Institute which found that among the 60 human cancer lines in which survivin is overexpressed, breast cancer is one of those with the highest levels, as well as lung cancer and CSCs [7]. The cancer lines used are breast (MCF-7) and cervical (HeLa) cancer, so the survivin promoter is a great candidate. Moreover, it should be noted that survivin is also overexpressed in CSCs, being this cell type the main target of our therapy [6].

The suicide genes we chose in this research are *hokD* and *IdrB.* When these genes are expressed in the cell, proteins of 52 and 35 amino acids, respectively, are obtained. These sizes are a great advantage compared to larger toxins such as diphtheria toxin A [23], with 535 amino acids; or stertolysin O, with 571 amino acids, used in a study by Wan Seok Yang et al. on suicide cancer gene therapy with this toxin [9], in which they obtained more than 70-90% reduction of cell viability *in vitro* in some cancer cell lines and *in vivo* in cervical carcinoma, but without the use of the Tet-On System or cancer/tissue-specific promoter. **Because of this size difference, these genes could be safer and more effective as gene therapy.**

In the present report, we studied the effect of *hokD* and *IdrB* as suicide genes under the control of the TRE3G-Dox promoter induced with Dox, which is not a toxic antibiotic [24] in HeLa and MCF-7 CSCs as three dimensional cultures and *in vivo* using HeLa CSCs in BALB/c mice. HeLa and MCF-7 CSCs transfected with *hokD* show a percentage inhibition of proliferation of approximately 100% in both cell lines at the end of the experiment; whereas HeLa and MCF-7 transfected with *IdrB* show between 61.64% and 23.46% inhibition, respectively, at the end of the experiment. Although the percentage of inhibition of MCF-7 transfected with *IdrB* seems to be insufficient, ATPlite levels indicate 72.07% cell death compared to the control. We can compare the results obtained with those presented in the study by Senthilkumar Kalimuthu et al [**22**], in which they used the Tet-On system to express HSV1-sr39TK in combination with MSCs in colon cancer cells. In it, they measured proliferation as a function of luciferase activity and represented it as relative photons/second (p/s) (%). Thus, by administering 1 µM of ganciclovir after 48 hours of co-cultured MSCs and CT26/Rluc in this assay, they obtained a 35% relative p/s in relation with no ganciclovir administration. This could be translated into approximately 65% inhibition of proliferation. This result is similar to the one presented here in HeLa and MCF-7 CSCs transfected with *hokD*; but superior to HeLa and MCF-7 CSCs transfected with *IdrB.* However, it is necessary to emphasize that the present invention shows results obtained in 3D cultures. If we consider the results obtained in previous works, in which we found a percentage of inhibition of proliferation in MCF-7-/drB-Dox spheroids after 15 days of treatment around >70% [12] the values of area, roundness and solidity of these 3D cultures show different results for each suicide gene used in this work. In the case of CSCs transfected with *hokD,* the spheroid was progressively disintegrating, decreasing the measured parameters compared to the control. In the case of CSCs transfected with *IdrB,* the area value remains significantly lower than those of the control, while the roundness and solidity values remain as those of the control. These results seem to indicate the actual effect of each gene. ***hokD* is responsible for cell death and spheroid destruction; while *IdrB* causes growth inhibition as well as cell death, which was corroborated by ATPlite levels.**

On the other hand, a tumor marker is a biomarker found in blood, urine or body tissues that may be elevated by the presence of one or more types of cancer. The ideal tumor marker should be specific and sensitive for detecting small tumors to allow early diagnosis or aid in detection. Few markers are specific for a single tumor. Hormone receptors (ER and PR), human epidermal growth factor receptor 2 (HER2), and proliferation-related genes are the main drivers of classification in many of the gene expression profiling tests for breast cancer. The bacterial toxin HokD induces suppression of ER and PR expression, thus representing a potential strategy to combat tumor cells as these receptors are related to cell growth and proliferation. Furthermore, this gene could combat resistance to endocrine therapy in ER+ breast cancer. However, *IdrB* only reduces the expression of PR and not ER. In addition, mutant p53 is known to increase cancer progression and malignancy. Immunocytochemical study showed a decrease of p53 in MCF, suggesting that ***hokD* and *IdrB* genes are inducing** a **reduction of the mutated gene and thus a better prognosis.**

In addition, in *vivo* experiments were performed in these investigations. A 91.6% and 95.7% inhibition of proliferation was obtained in HeLa CSCs expressing *hokD* and *IdrB,* respectively, at 35 days. The mean tumor volume after 35 days of treatment was reduced by 80% and 98% in HeLa CSCs expressing *hokD* and *IdrB,* respectively, compared with the control group. In addition, the treated mice showed no side effects. In previous studies [40] it seems that a reduction of tumor growth in mice is also obtained; although the percentage of tumor volume reduction is not indicated, but, on the contrary, treated mice lost weight.

Given these results, not only the potency of the Tet-On system and the TRE3G-Dox promoter is confirmed, but also better results are obtained in combination with *hokD* and *IdrB.*

Experiments with the cancer-specific survivin promoter with differentiated HeLa and MCF-7 cells cultured in monolayer and transfected with *hokD* and *IdrB* genes show a significant inhibition of cell proliferation, being more than 95% inhibition with *hokD* and *IdrB* genes in both cell lines at the end of the experiment; with a progressive increase of the percentage. In comparison, other tissue-specific promoters, such as, for example, the osteonectin promoter (hON-522E), show better results. **[18]** which controls tyrosine kinase (TK) expression in androgen-independent human prostate cancer (PC3M) transfected by adenoviral vectors. In this research they obtained that PC3M infected with Ad-522E-TK at Multiplicity of Infection (MOI) values of 10, 30 and 100, and administered with ganciclovir, presents survival percentages around 50, 20 and 10%. In other words, the percentages of inhibition of proliferation are 50, 80 and 90%, respectively. The use of the mNUS promoter **[19]** controlling the expression of a recombinant cytosine deaminase (RCD) [**25**] obtained approximately 60% of dead Tera-1 cells transfected with mNUS-RCD +5-FC (a prodrug transformed by CD into a toxic compound). Another case is that of the TTS system controlling the expression of the proapoptotic X protein-associated gene Bcl-2 (Bax) [**20**] used to transfect lung adenocarcinoma cells (PC3) where the sub-G1 DNA content increases by 42.16%, suggesting that Ad-TTS/Bax induces cell death due to Bax expression. Furthermore, comparisons can be made with other studies where the survivin promoter (Surp1430) was used controlling diphtheria toxin A (DTA) expression [**26**]. In this case, the reduction in viability of malignant cells was 42 % and 92 % with 0.1 and 0.3 µg of the recombinant lentiviral vector used to transfect the Surp1430-DTA construct, without affecting normal cells.

As can be seen, *hokD* and *IdrB* under the control of the survivin promoter seem to produce more efficient results in **two-dimensional cultures** than other cancer/tissue-specific promoters or even other suicide genes controlled by the survivin promoter. Previously, about 70% inhibition of proliferation was described in MCF-7 and HCT-116 cells transfected with *IdrB* but under the control of the TRE3G-Dox promoter. This gives us an idea of the promising specific effect of the survivin promoter as a potential suicide gene therapy against cancer.

In **3D cultures,** we performed the experiments with spheroids of differentiated HeLa and MCF-7 cells and HeLa CSCs spheroids. The results showed a progressive inhibition of proliferation, reaching after 17 days between 60% inhibition of proliferation in spheroids of both HeLa and MCF-7 differentiated cells transfected with *hokD*; 92.82% in spheroids of HeLa differentiated cells transfected with *IdrB*; and 74.65% in spheroids of MCF-7 differentiated cells transfected with *IdrB.* Comparing with the results obtained in the previous work on *IdrB* **[12],** a similar effect can be observed with *hokD*; however, in this case, the survivin promoter and another suicide gene allow specific action with the same inhibition results. On the other hand, the results obtained with spheroids of differentiated HeLa cells transfected with *IdrB* indicate a greater effect, and can be considered as one of the best combinations in these cases.

Although, the explicit effect of each gene seemed to be clear in CSCs transfected with *hokD* under the control of the TRE3G-Dox promoter; in the case of the survivin promoter, the effectiveness is almost complete with *hokD* and *IdrB.* On these occasions, HeLa CSCs transfected with *hokD* at 8 days show 37.87% inhibition of proliferation, reaching 95.5% at 10 days; whereas HeLa CSCs transfected with *IdrB* at 8 days show 24.04% inhibition of proliferation, reaching 83.99% at 10 days. The results corroborate the specificity of the survivin promoter in CSCs, since these 3D cultures show a greater inhibition of proliferation than those of differentiated cells.

Moreover, the values of area, roundness and robustness present a similar pattern to those of CSCs transfected with *hokD* under the control of the TRE3G-Dox promoter, which allows us to confirm the specific effect of each suicide gene used in this work. However, in HeLa and MCF-7 CSCs transfected with *IdrB* under the control of the TRE3G-Dox promoter, these parameters appear to be the same as those of the control, and may be confounded about the antitumor effect of this gene. However, it can be refuted with ATPlite levels as discussed above, presenting 99.16% and 72.07% cell death in HeLa and MCF-7 CSCs transfected with *IdrB* under the control of the TRE3G-Dox promoter.

As mentioned above, the safety in non-tumor cells was demonstrated in follicular cells, and the results determine that, despite being transfected with suicide genes under the control of the survivin promoter, follicular cells proliferate about >1.5-fold more than the control (143.76% and 174.12% in follicular cells transfected with *hokD* and *IdrB,* respectively). It means that ***hokD* and *IdrB* under the control of survivin promoter alone has an antitumor effect on cancer cells, and could be a safer and less toxic option in cancer gene therapy.** Moreover, the more specific the cancer treatment was, the less side effects the treatment has. Thus, and taking into account that *hokD* and *IdrB* under the control of the survivin promoter **do not affect follicular cells,** one of the most remarkable side effects of radio and chemotherapy, **hair loss, could be avoided with this specific therapy.**

Finally, in order to learn the effect of this particular suicide gene therapy in a whole organism, *in vivo* experiments were performed in mice. Mice with HeLa CSCs tumors were injected intratumorally with plasmid containing *hokD* or *IdrB* under the control of the survivin promoter. After 32 days of treatment, the mice experienced a decrease in mean tumor volume, being about 65% less than the control group in HeLa CSCs treated with *hokD* and *IdrB* under the control of the survivin promoter, presenting an inhibition of proliferation of 76.21% and 79.3% at 32 days, respectively. Other previously discussed work with other cancer/tissue-specific promoters [**18**], including the survivin promoter that controls the expression of other genes [**25**], also show good results *in vivo.* In the case of Ad-522E-TK combined with GCV in a PC3M subcutaneous xenograft model in nude mice [**18**], tumor volume decreases by approximately 80% after 35 days of treatment. While the xenograft model of nude mice with ZR7530 breast cancer tumor treated with the lentiviral vector Surp1430-DTA [**26**] appears to reduce tumor volume by more than approximately 95%. While these other results appear to be more effective in decreasing tumor volume, in no case were the tumors eradicated in their entirety. Despite this, the mice **do not present any side effects such as metastasis, hair loss, weight loss, among others,** in any of these cases; contrary to what happens in other cases [**22**], where the mice lost weight during treatment.

Compiling all the results obtained, ***hokD* and *IdrB* have a cancer-specific effect on HeLa and MCF-7 cell lines being controlled by the survivin promoter, showing better results in two-dimensional cultures than other investigations.** In particular, we can corroborate with results in 3D cultures what was previously discussed about the **individual effect of *hokD*, which may be cell death, and *IdrB,* which may be inhibition of proliferation.** Therefore, this research opens the door to the possibility of **using the combination of *hokD* and/or *IdrB* genes as suicide gene therapy in breast and cervical cancer, due to the specific and potent antitumor effect *in vitro* and *in vivo,* and avoiding the side effects** of the treatments recently used for these cancers due to their selectivity and high antiproliferative effect only in tumor cells without affecting the rest of the organism.

### References:

1. Kaufmann, K.B., et al., Gene therapy on the move. EMBO Mol Med, 2013. 5(11): p. 1642-61.
2. Navarro, S.A., et al., Cancer suicide gene therapy: a patent review. Expert Opin Ther Pat, 2016. 26(9): p. 1095-104.
3. Montano-Samaniego, M., et al., Strategies for Targeting Gene Therapy in Cancer Cells With Tumor-Specific Promoters. Front Oncol, 2020. 10: p. 605380.
4. Fukuda, S. and L.M. Pelus, Survivin, a cancer target with an emerging role in normal adult tissues. Mol Cancer Ther, 2006. 5(5): p. 1087-98.
5. Blum, B., et al., The anti-apoptotic gene survivin contributes to teratoma formation by human embryonic stem cells. Nat Biotechnol, 2009. 27(3): p. 281-7.
6. Warrier, N.M., P. Agarwal, and P. Kumar, Emerging Importance of Survivin in Stem Cells and Cancer: the Development of New Cancer Therapeutics. Stem Cell Rev Rep, 2020. 16(5): p. 828-852.
7. Shojaei, F., et al., Trace of survivin in cancer. Eur J Cancer Prev, 2019. 28(4): p. 365-372.
8. Shapira, A. and I. Benhar, Toxin-based therapeutic approaches. Toxins (Basel), 2010. 2(11): p. 2519-83.
9. Yang, W.S., et al., Suicide cancer gene therapy using pore-forming toxin, streptolysin O. Mol Cancer Ther, 2006. 5(6): p. 1610-9.
10. Boulaiz, H., et al., gef gene expression in MCF-7 breast cancer cells is associated with a better prognosis and induction of apoptosis by p53-mediated signaling pathway. Int J Mol Sci, 2011. 12(11): p. 7445-58.
11. Ramirez, A., et al., Enhancement of Tumor Cell Death by Combining gef Gene Mediated Therapy and New 1,4-Benzoxazepin-2,6-Dichloropurine Derivatives in Breast Cancer Cells. Front Pharmacol, 2018. 9: p. 798.
12. Jimenez-Martinez, Y., et al., LdrB Toxin with In Vitro and In Vivo Antitumor Activity as a Potential Tool for Cancer Gene Therapy. Cancers (Basel), 2019. 11(7).
13. Steif, A. and I.M. Meyer, The hok mRNA family. RNA Biol, 2012. 9(12): p. 1399-404.
14. Dawood, S., L. Austin, and M. Cristofanilli, Cancer stem cells: implications for cancer therapy. Oncology (Williston Park), 2014. 28(12): p. 1101-7, 1110.
15. Nassar, D. and C. Blanpain, Cancer Stem Cells: Basic Concepts and Therapeutic Implications. Annu Rev Pathol, 2016. 11: p. 47-76.
16. Marusyk, A. and K. Polyak, Tumor heterogeneity: causes and consequences. Biochim Biophys Acta, 2010. 1805(1): p. 105-17.
17. Duzgunes, N., Origins of Suicide Gene Therapy. Methods Mol Biol, 2019. 1895: p. 1-9.
18. Sung, S.Y., et al., Co-Targeting Prostate Cancer Epithelium and Bone Stroma by Human Osteonectin-Promoter-Mediated Suicide Gene Therapy Effectively Inhibits Androgen-Independent Prostate Cancer Growth. PLoS One, 2016. 11(4): p. e0153350.
19. Kuzmin, D., et al., Novel strong tissue specific promoter for gene expression in human germ cells. BMC Biotechnol, 2010. 10: p. 58.
20. Fukazawa, T., et al., Pulmonary adenocarcinoma-targeted gene therapy by a cancer- and tissue-specific promoter system. Mol Cancer Ther, 2007. 6(1): p. 244-52.
21. Randolph, L.N., et al., An all-in-one, Tet-On 3G inducible PiggyBac system for human pluripotent stem cells and derivatives. Sci Rep, 2017. 7(1): p. 1549.
22. Kalimuthu, S., et al., Regulated Mesenchymal Stem Cells Mediated Colon Cancer Therapy Assessed by Reporter Gene Based Optical Imaging. Int J Mol Sci, 2018. 19(4).
23. Parveen, S., W.R. Bishai, and J.R. Murphy, Corynebacterium diphtheriae: Diphtheria Toxin, the Tox Operon, and Its Regulation by Fe2(+) Activation of apo-DtxR. Microbiol Spectr, 2019. 7(4).
24. VanderVeen, N., et al, Effectiveness and preclinical safety profile of doxycycline to be used "off-label" to induce therapeutic transgene expression in a phase I clinical trial for glioma. Hum Gene Ther Clin Dev, 2013. 24(3): p. 116-26.
25. Erbs, P., et al., In vivo cancer gene therapy by adenovirus-mediated transfer of a bifunctional yeast cytosine deaminase/uracil phosphoribosyltransferase fusion gene. Cancer Res, 2000. 60(14): p. 3813-22.
26. Lin, B., et al., Use of a Novel Integrase-Deficient Lentivirus for Targeted Anti-Cancer Therapy With Survivin Promoter-Driven Diphtheria Toxin A. Medicine (Baltimore), 2015. 94(31): p. e1301.

## Claims

1. A genetic construct comprising a nucleotide sequence corresponding to a gene expression system or vector comprising at least one RNA or DNA polynucleotide of the *hokD* gene and of a proliferation inhibitor gene, operatively linked to at least one promoter directing transcription of said nucleotide sequence, and to other sequences necessary or appropriate for transcription and its regulation suitable in time and place for transcription *in vitro* or *in vivo.*

2. A genetic construct according to the preceding claim wherein the proliferation inhibitor gene is *IdrB.*

3. A genetic construct according to any one of claims 1 to 2 wherein the transcription-directing promoter is selected from the list consisting of telomerase promoter, epidermal growth factor receptor promoter, transferrin receptor promoter, carcinoembryonic antigen promoter and survivin promoter or any combination thereof.

4. A genetic construct according to the preceding claim wherein the promoter directing transcription is the survivin promoter.

5. A cell comprising a genetic construct according to any one of claims 1 to 4.

6. A composition comprising:
a) a genetic construct according to according to any one of claims 1 to 4, and/or
b) a cell according to claim 5.

7. The genetic construct according to any one of claims 1 to 4, the cell according to claim 5, and/or the composition according to claim 6, for use as a medicament.

8. The genetic construct according to claim 1 to 3, the cell according to claim 4, and/or the composition according to claim 5 for the prevention, amelioration, alleviation or treatment of a proliferative disease.

9. The genetic construct, cell and/or composition for use according to claim 8, wherein the proliferative disease is cancer.

10. The genetic construct, cell and/or composition for use according to the preceding claim, wherein the cancer is **characterized by** presenting cancer stem cells.

11. The polynucleotide, genetic construct, cell and/or composition for use according to claims 9 to 10, wherein the proliferative disease is breast cancer.

12. The polynucleotide, genetic construct, cell and/or composition for use according to claims 9 to 10, wherein the proliferative disease is cervical cancer.

13. The polynucleotide, genetic construct, cell and/or composition for use according to claim 11, in combination with any of the drugs selected from the list consisting of: Trastuzumab, Trastuzumab deruxtecan, Pertuzumab, Ado-trastuzumab emtansine, Sacituzumab govitecan, Tucatinib, Neratinib, Lapatinib, Palbociclib, Ribociclib, Abemaciclib, Alpelisib, Everolimus, Olaparib, Talazoparib and Atezolizumabo any combination thereof.

14. The polynucleotide, genetic construct, cell and/or composition for use according to claim 12, in combination with any of the drugs selected from the list consisting of: Cisplatin, Bevacizumab, Pembrolizumab, Carboplatin, Tisotumab vedotin, Gemcitabine, Ifosfamide, Irinotecan and Paclitaxel any combination thereof.
